# EUROPEAN PATENT APPLICATION

(11) **EP 1 990 347 A1**
(43) Date of publication of application: **12.11.2008**
(21) Application number: 07009168.1
(22) Date of filing: 07.05.2007
(51) Int. Cl.: C07F 15/00, C12Q 1/28, C12Q 1/54, C12Q 1/60

(54) **Quantification of hydrogen peroxyde by triggered reconstitution of horseradish peroxidase using a modified hemin**

(71) Applicant: Universität Heidelberg, 69117 Heidelberg (DE)
(72) Inventor: Krämer, Roland, 69126 Heidelberg (DE); Pavlov, Valery, 2009, San Sebastian (ES)
(74) Representative: Müller-Boré & Partner Patentanwälte

(57) **Abstract**

The present invention relates to specific hemin derivatives, a kit comprising these hemin derivatives in combination with apo-horseradish peroxidase (apo-HRP), and an assay method for detecting and/or quantifying an analyte in a biological sample which comprises detecting hydrogen peroxide generated in the presence as opposed to the absence of said analyte by bringing said sample into contact with such a kit.

## Description

The present invention relates to specific hemin derivatives, a kit comprising these hemin derivatives in combination with apo-horseradish peroxidase (apo-HRP), and an assay method for detecting and/or quantifying an analyte in a biological sample which comprises detecting hydrogen peroxide generated in the presence as opposed to the absence of said analyte by providing said sample with such a kit.

The fast and sensitive quantification of H₂O₂ is of significant interest in analytical chemistry and biochemistry as well as in diagnostic settings. In particular, in commercial ELISA kits using glucose oxidase (GO)- antibody conjugates, the analyte is indirectly quantified by detecting the amount of H₂O₂ which is produced by immmobilized GO during catalytic oxidation of glucose by O₂. In this context, a fluorimetric assay using the HRP catalyzed oxidation of the relatively expensive fluorogenic substrate Amplex Red by H₂O₂ has evolved as a standard detection method with a 50 nM sensitivity limit in microplate wells (i.e. 5 pMol in 100 µL reaction solution), whereas the majority of current ELISA systems are built on peroxidase labelled immunoconjugates and detection of the peroxidase activity by chromogenic substrates such as TMB and ABTS.

However, there is still a need for more sensitive assays concerning the quantification of H₂O₂. In particular, there is still a demand for a simple, rapid and sensitive method of quantifying the level of a certain kind of substance including glucose and cholesterol, etc., in clinical samples such as patient's plasma. If a highly sensitive assay method for hydrogen peroxide becomes available, it will be possible to conveniently quantify such a substance in a sample by way of detecting a trace amount of hydrogen peroxide generated by an enzyme such as an oxidase on said substance.

Accordingly, the object underlying the present invention is to provide a new reagent or system being able to fastly and sensitively quantify H₂O₂ where said H₂O₂ is either present in a sample to be assayed or is generated *in situ* in a sample from a substance to be monitored/quantified by the action of an enzyme, preferably an oxidase, and the detected H₂O₂ level is used as the measure for the amount of the substance to be quantified.

The solution to the above technical problem is achieved by providing the embodiments characterized in the claims.

In particular, in a first aspect of the present invention there is provided a hemin derivative which has the following structure: 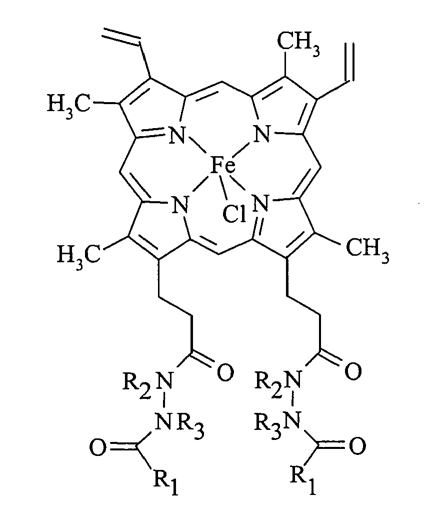 wherein
R₁ represents hydrogen, -ORₐ, -SRₐ, -NRₐR_{b}, a C₁₋₈ straight chain or branched chain alkyl residue, an unsubstituted or C₁₋₈ alkyl- or C₁₋₈ alkoxy-substituted C₆₋₁₀ aryl residue, particularly phenyl or tolyl, or a C₆₋₁₀ aralkyl residue, particularly benzyl, with Rₐ and R_{b} being independently from each other selected from hydrogen, a C₁₋₈ straight chain or branched chain alkyl residue, a C₆₋₁₀ aryl residue, particularly phenyl, or a C₆₋₁₀ aralkyl residue, preferably benzyl, and
R₂ and R₃, independently from each other, represent hydrogen, a C₁₋₈ straight chain or branched chain alkyl residue, a C₆₋₁₀ aryl residue, particularly phenyl, or a C₆₋₁₀ aralkyl residue, preferably benzyl.

Preferably, R₁ represents a C₁₋₈ straight chain alkyl residue, more preferably methyl. Preferably, R₂ and R₃ represent hydrogen.

In a second aspect of the present invention there is provided a kit comprising
(i) a hemin derivative as defined above, and
(ii) apo-horseradish peroxidase (apo-HRP).
   Preferably, such a kit further comprises:
(iii) a substrate selected from the group consisting of chromogenic, fluorogenic and luminescent substrates.

The term "kit" as used refers to a packaged set of related components.

In another aspect of the present invention there is provided an assay method for detecting and/or quantifying an analyte in a biological sample which comprises detecting hydrogen peroxide generated in the presence as opposed to the absence of said analyte by bringing said sample into contact with such a kit. Accordingly, the present invention includes a method for determining the presence or absence of hydrogen peroxide in a sample, wherein the method comprises
- contacting the sample with the above kit comprising constituents (i), (ii) and (iii),
- incubating the sample for a sufficient amount of time to prepare an incubated sample, wherein H₂O₂, if present, deprotects the chemically blocked hemin derivative according to the present invention and, thus, activates the enzyme horseradish peroxidase (HRP) which, in turn, catalyzes the conversion of the respectively selected substrate (iii), i.e. a horseradish peroxidase (HRP) substrate, into a detectable molecular compound,
- depending on the substrate chosen and its resulting detectable compound, optionally illuminating the incubated sample with an appropriate wavelength, and
- observing the thus treated sample whereby the presence or absence of hydrogen peroxide in the sample is determined.

The present invention describes a new system for the colorometric quantification of H₂O₂ at a detection limit 50 nM and 50 fMol, respectively, without the need of specialized instrumentation. The system or reagent, respectively, is a mixture of commercially available apo-horseradish peroxidase and a newly synthesized bis(N-acylhydrazido)hemin as given above. The present invention is based on newly synthesized hemin di(N,N'-acylhydrazide) derivatives and resides in that the reconstitution of these compounds with apo-horseradish peroxidase yields active enzyme only in the presence of hydrogen peroxide. H₂O₂ deprotects the chemically blocked hemin derivatives according to the present invention and, thus, activates the enzyme horseradish peroxidase (HRP) which, in turn, catalyzes the conversion of a respectively selected substrate into a detectable molecular compound. This oxidative reconstitution can advantageously be applied for the ultra-sensitive colorimetric detection of e.g. hydrogen peroxide and glucose oxidase. In particular, the present invention can suitably used in assays such as ELISA where in the detection step antibodies are conjugated with an oxidase. Accordingly, the hydrogen peroxide to be detected can be the product formed by an enzymatic reaction like the oxidation of a substrate using an oxidase. Said oxidase can be selected from the group consisting of glutamate oxidase, amine oxidase, choline oxidase, cholesterol oxidase, galactose oxidase, xanthin oxidase, uricase oxidase, pyruvate oxidase, glycerin-3-phosphate oxidase, acyl Co A oxidase, glycerol oxidase and glucose oxidase.

Advantageously, the present invention allows to use existing photometric equipment and well established peroxidase detection techniques to perform sensitive ELISA assays based on e.g. GO-antibody conjugates. Detection of H₂O₂ relies on the triggered generation of peroxidase activity from an enzymatically inactive mixture of anyone of the above specified hemin derivatives, particularly bis(N-acetylhydrazido)hemin and apo-horseradish peroxidase. Presumably, the peroxidase activity is triggered by H₂O₂-dependent oxidative conversion of bis(N-acetylhydrazido)hemin into hemin, followed by reconstitution with apo-HRP into active HRP.

Subsequently, HRP can be detected by diluting the reaction solution with a standard ELISA assay solution, for example using the chromogenic substrate tetramethylbenzidine (TMB) and detecting the generation of yellow (blue, respectively) color of oxidized TMB by photometry or by naked eye. Assuming that the observed peroxidase activity origins from reconstituted HRP, 50 nM H₂O₂ trigger the formation of about 0.1 nM active HRP after 40 min incubation time, and HRP strongly amplifies the signal by conversion of TMB into its colored oxidation product (about 200 nM in 3 min). By native HRP alone, the limit of H₂O₂ detection using the substrate TMB is 1 µM, i.e. the bis(N-acetylhydrazido)hemin/apo-HRP reagent improves the sensitivity by a factor of 20.

The present invention is not limited to the TMB assay but allows to improve significantly the sensitivity of H₂O₂ detection by any method based on HRP-catalyzed oxidation of chromogenic (for example TMB, ABTS, guajacol), fluorogenic (for example Amplex Red) or luminescent (luminol) substrates. In a more general context, the present invention describes the first example of analyte-triggered activation of the chemically blocked hemin cofactor, followed by large signal amplification using the catalytic power of reconstituted HRP. This reactivity-based detection principle should be applicable to development of simple but ultrasensitive chromogenic, fluorogenic and luminescent assays for any analyte which is capable of deblocking carboxy-protected hemin cofactor.

Chemical derivatives of hemin have been an object of investigations for a long time. Numerous hemin derivatives were reported such as esters of hemin, hemin lacking carboxyl groups, hemin with acetyl groups instead of vinyl ones, hemin with hydrogen atoms substituting vinyl groups, proto-, hemato-, meso-, deutero-, diacetyldeutero-, dipropenyldeutero-, and dibutenyldeuterohematins, hemin amides with amino acids, 2-formyl-4-vinyl-deuterohemin- and 2-vinyl-4-formyl deuterohemin-substituted hemins, 2-formyl-4-vinyldeuterohemin, 2-vinyl-4-formyldeuterohemin, 2,4-dimethyldeuterohemin, and 2,4-diacetyldeuterohemin, deutero hemin, 2,4-diacetyldeuterohemin, 2-vinyl-4-deuterohemin. Studies of reconstitution of the above mentioned hemin derivatives with apo-horseradish peroxidase demonstrated that the side chains in positions 2 and 4 of the porphyrin ring are not important for the catalytic activity of reconstituted peroxidase. At the same time the presence of carboxylic groups at positions 6 and 7 determines the enzymatic activity. For example, apo peroxidase reconstituted with monomethyl ester of hemin demonstrates only 20% activity of the native enzyme and dimethyl ester shows no activity at all.

Hydrazide derivatives of hemin have not been reported yet. It is known that chemical oxidation of hydrazide group yields carboxylic acid, therefore the phenylhydrazide group was extensively used as a protective group in peptide synthesis. The methods for the deprotection of carboxyl group rely on the chemical oxidation of phenylhydrazine to unstable phenyldiimide followed by a spontaneous decomposition of the latter. As respective oxidants potassium permanganate, ferric chloride, iodine, bromosuccinimide, manganese dioxide, lead and copper acetates were employed.

The serious drawback of these oxidizing agents is the necessity to use harsh conditions for the deblocking reaction leading to undesirable oxidation of some residues. It was shown that horse radish peroxidase selectively removes phenylhydrazine protecting group under mild conditions. Previous reports demonstrated that peroxidase is able to use a number of hydrazine derivatives as substrates including isonicotinic acid hydrazide, phenylethyl-, ethyl-, and methylhydrazine, 3-aminophthalhydrazide (known as luminol), isoluminol, N-(6-aminobutyl)-N-ethyl isoluminol, and 7-dimethylaminonaphthalene-1,2-dicarboxylic acid hydrazide.

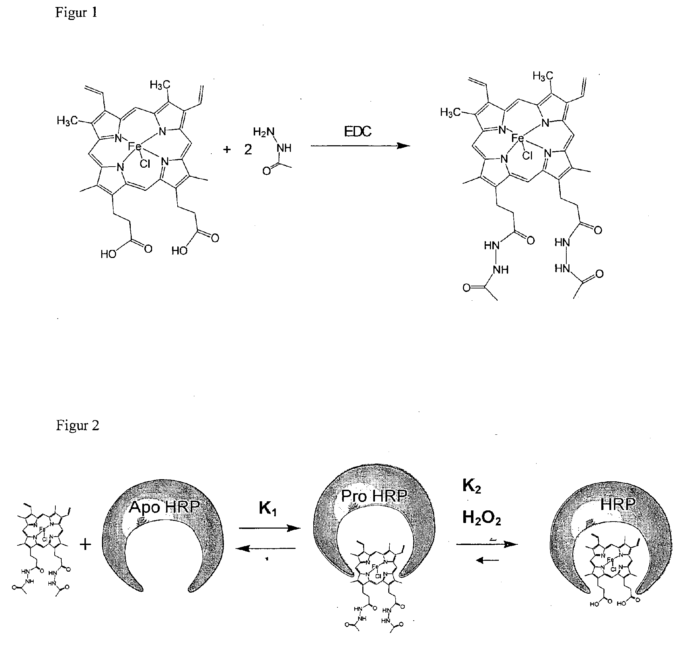

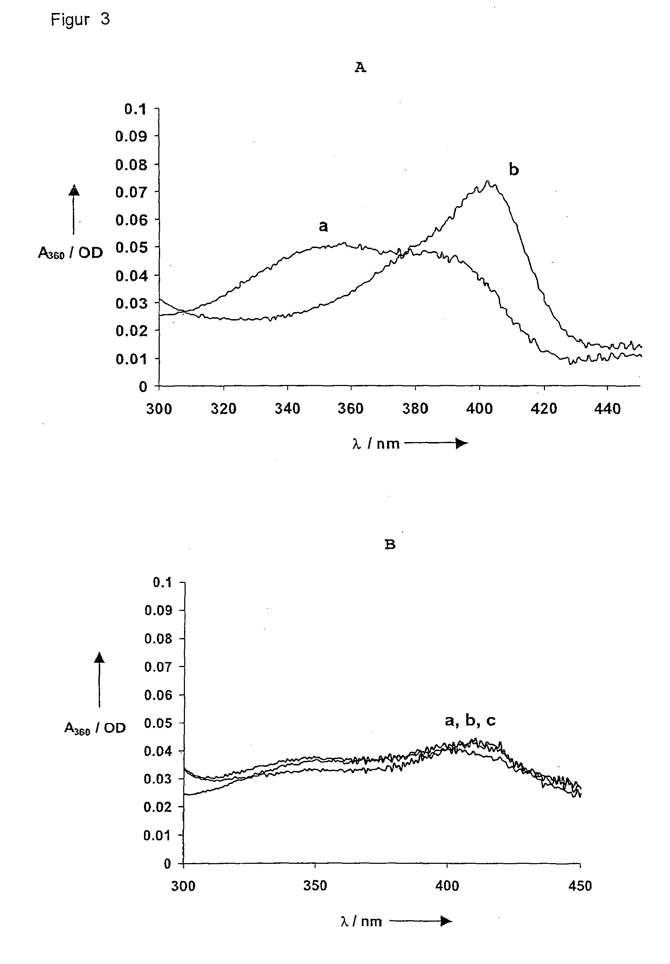

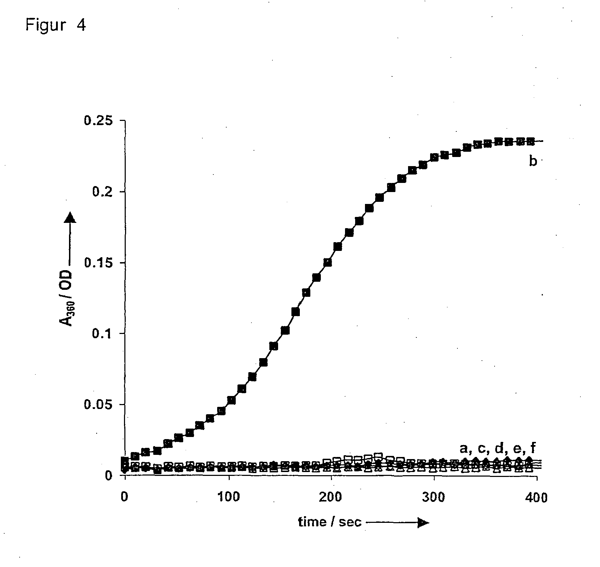

The synthetic route to hemin di(N,N'-acyl-hydrazide) derivatives like hemin di(N,N'-acetyl-hydrazide) is outlined in Figure 1. Use of 1-(dimethylaminopropyl)-3-ethylcarbodiimide hydrochloride (EDC) as a coupling agent allows to synthesize the desired material with high yield of c.a. 90% under mild conditions. The compound shows high stability during chromatographic separation and storage contrary to fears that this material could be unstable due to possible oxidation by air oxygen mediated by the hemin moiety *via* intramolecular mechanism. In general, all 1,2-diacylated hydrazines demonstrate chemical inertness and are resistant to the action of strong oxidants. They can be oxidized only after a preliminary hydrolysis in 2 N sulphuric acid at 100°C. The UV/VIS absorption spectrum of hemin di(N,N'-acetyl-hydrazide) is different from that of hemin. The shape of absorption peaks is not changed upon addition of excessive amount of apo-horseradish peroxidase into a solution of hemin di(N,N'-acetyl-hydrazide). This behaviour is not similar to that of hemin, which in the presence of apo enzyme changes its UV/VIS absorption signature due to rapid reconstitution with the latter under equal experimental conditions (cf. Figure 3). This confirms that hemin di(N,N'-acetyl-hydrazide), like all other hemins with modified carboxylic groups at positions 6 and 7, does not form a stable complex with apo-horseradish peroxidase. In addition, the mixture of apo-horseradish peroxidase and di(N,N'-acetyl-hydrazide) after 10 min preincubation does not show any peroxidase activity in a colorimetric activity assay based on oxidation of a colorogenic substrate like 3,3',5,5'-tetramethylbenzidine dihydrochloride (TMB) by hydrogen peroxide (cf. Figure 4, curve (a)). When preincubation is carried out in the presence of hydrogen peroxide a significant increase in peroxidase activity can be detected after 10 min starting from the moment of addition of H₂O₂ (cf. Figure 4, curve (b)). It should be emphasized that this increase in peroxidase activity is not an artefact caused by the rise of peroxide concentration in the assay mixture containing TMB and 0.17 mM H₂O₂, because, firstly, peroxidase activity increases with time as one can see when comparing curves (b) and (c), secondly, injection of the sample solution into the assay solution leads to 100 fold dilution of pre-incubated mixture containing hemin di(N,N'-acetyl-hydrazide) and 0.17 mM H₂O₂. Control experiments performed with mixtures preincubated during 10 min containing only two components of three possible ones, i.e. hemin di(N,N'-acetyl-hydrazide) plus H₂O₂, apo-horseradish peroxidase plus H₂O₂, demonstrated the absence of peroxidase activity increase during incubation (cf. Figure 4, curves (d), (e)). Another experiment in which hemin di(N,N'-acetyl-hydrazide) was preincubated for 10 min in the presence of H₂O₂ and apo peroxidase was added later, just before the peroxidase assay, showed no increase in peroxidase activity too (cf. Figure 4, (f)). These experimental data prove that hemin di(N,N'-acetylhydrazide) cannot be oxidized by hydrogen peroxide in the absence of apo-horseradish peroxidase. All three components, i.e. apo-horseradish peroxidase, hemin di(N,N'-acetyl-hydrazide) and H₂O₂ are essential for generation of peroxidase activity in the system.

Without being bound thereto, the following mechanism outlined in Figure 2 is proposed to explain said experimental results. The equilibrium between free hemin di(N,N'-acetyl-hydrazide) and free apo-H R P on the left side and the complex of hemin di(N,N'-acetyl-hydrazide) and apo-HRP (pro-HRP) is significantly shifted to the left side due to the modification of carboxylic groups at positions 6 and 7 by hydrazide moieties. Addition of H₂O₂ leads to irreversible oxidation of hydrazide groups in pro-H R P, catalyzed by the proteic shell, and formation of full functional peroxidase. Thus, the first example of oxidative reconstitution *in vitro* has been discovered. It can be speculated that in *vivo* similar redox reconstitutions also can play a role during self-assembling of enzymes and they constitute an important part of enzyme activation mechanism triggered by an enzyme substrate.

The influence of H₂O₂ concentration on the rate of peroxidase activation under optimized experimental conditions is presented in Figures 5, 6, and 7.

Figure 5 illustrates measurements of peroxidase activity after preincubation for 40 min. with varying concentrations of H₂O₂. Development of generated peroxidase activity with time for different concentrations of apo-horseradish peroxidase is presented in Figure 6.

Figure 7 gives the dependence of peroxidase activity, derived from slopes of lines in Figure 5, on the concentration of H₂O₂. Activation rate was calculated by measuring initial rate of TMB oxidation for each concentration of H₂O₂ in incubation mixtures every 10 min.

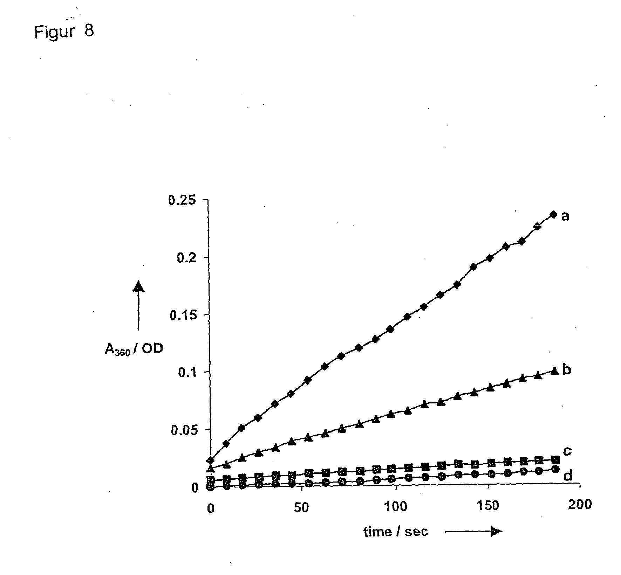

In the course of the present invention, it has been found that the system can be applied for colorimetric detection of H₂O₂ using for example TMB as substrate with detection limit not worse than 50 nM under physiological conditions. The sensitivity to hydrogen peroxide of such a colorimetric system according to the present invention is comparable with that of the commercially available fluorometric system based on the oxidation of fluorogenic substrate 10-acetyl-3,7-dihydroxyphenoxazine (Amplex Red). The oxidative reconstition approach as adopted by the present invention can be easily applied for detection of any enzyme generating hydrogen peroxide, for example glucose oxidase. The effect of glucose oxidase concentration in preincubation mixture on the resulting peroxidase activity is shown in Figure 8. The detection limit was 40 µU/ml of glucose oxidase. Amplification cascade based on oxidative reconstitution of HRP triggered by glucose oxidase can find broad application in immuno assays relying on glucose oxidase immunoconjugates as labels of a recognition event. The majority of current ELISA systems are build on the basis of peroxidase labelled immunoconjugates and detection of peroxidase activity by colorogenic substrates such as TMB and ABTS. The introduction of the new concept for signal amplification based on triggered generation of peroxidase will allow to use existing equipment and well established peroxidase detection techniques to perform much more sensitive ELISA tests.

The following examples are given by way of illustration only and are not limiting the scope of the invention.

### Examples

### Materials:

Hemin chloride, Tris buffer, 5 M sodium chloride solution, EDTA tetrasodium salt, dimethylformamide, acetylhydrazide, 1-(dimethylaminopropyl)-3-ethylcarbodiimide hydrochloride (EDC), 3,3',5,5'-tetramethylbenzidine dihydrochloride (TMB), 2,2¹-azino-bis(3-ethylbenzthiazoline-6-sulphonic acid) (ABTS) were obtained from Sigma-Aldrich-Fluka (München, Germany). Apo-horseradish peroxidase was acquired from Calzyme (San Luis Obispo, CA, USA). Hydrogen peroxide solution was purchased from Schuster (Germany). HPLC quality water used during this work for the preparation of all aqueous solutions was obtained from VWR international bvbq/spr (Leuven, Netherlands).

### Preparation of hemin di(N,N'-acetyl-hydrazide):

Hemin chloride (50 mg, 76.7 µmol) and acetylhydrazide (47 mg, 0.634 mmol) were dissolved in 5 mL of dimethylformamide. Then, 1-(dimethylaminopropyl)-3-ethylcarbodiimide hydrochloride (67 mg, 0.35 mmol) was dissolved in 5 mL of dimethylformamide and added with stirring. The reaction mixture was left to stay at room temperature overnight. Then, 15 mL of methanol/chloroform (3:7) mixture was added to the reaction mixture and the products formed were separated by column chromatography (60x5 cm) on silica gel (Merck, 0.063-0.2 mm). The reaction products were eluted with 3:7 methanol/chloroform mixture. The second brown fraction was collected and evaporation of solvent in vacuum gave hemin di(N,N'-acetylhydrazide) with 90% yield.

Electrospray mass spectrometry was used to determine molecular weight of cationic form of the product (728.26 g/mole).

Electrospray mass spectrometry was performed on Q-TOF Ultima API Masspectrometer (Waters, Milford, USA) using 10⁻⁴ - 10⁻⁵ M solutions of modified hemin in acetonitrile with addition of tetrafluoroacetic acid.

### Oxidative reconstitution of hemin di(N,N'-acetyl-hydrazide)

Hemin di(N,N'-acetyl-hydrazide) was dissolved in DMF to obtain 6.5 mM solution. This DMF solution was added to a mixture containing 0.1 M Tris buffer (pH 7.5), 110 µM of EDTA, 0.26 M of sodium chloride, 0.86 µM of apo-horseradish peroxidase to obtain 2.33 µM of hemin di(N,N'-acetyl-hydrazide). Reconstitution was initiated by the addition of hydrogen peroxide solution of varying concentrations. Upon incubation during varying time intervals activity of resulting peroxidase was determined by UV spectroscopy.

### Detection of glucose oxidase:

The mixture described in the previous section was prepared with 72 mM glucose in addition. Reconstitution was initiated by the addition of glucose oxidase solution of varying concentrations and resulting peroxidase activity was determined by UV spectroscopy.

### Assays of peroxidase activity:

### Optimized colorimetric assay based on TMB

The assay was initiated by injecting a sample into a mixture containing 1 mM TMB and 2.3 mM hydrogen peroxide in 0.2 M sodium citrate buffer (pH 4.0) at room temperature. The factor of sample dilution in the assay mixture was 1:100. An increase in absorbance at 360 nm was followed.

Absorption measurements were recorded with UV/VIS spectrometer Spectra Fluor Plus integrated in Genesis Workstation (Tecan, Crailsheim, Germany) using standard 96 well plastic microplates Bio-One (Greiner,

The figures show the the following:
**Figure 1** shows the synthesis of hemin di(N,N'-acetyl-hydrazide).
**Figure 2** shows a proposed mechanism for the oxidative reconstitution of hemin di(N,N'-acetyl-hydrazide) with apo-HRP.
**Figure 3** shows **(A)** UV-visible absorption spectra of a) free hemin; b) hemin after 30 min incubation with apo-horseradish peroxidase; and **(B)** UV-visible absorption spectra of a) free hemin di(N,N'-acetyl-hydrazide); b) hemin di(N,N'-acetylhydrazide) after 30 min incubation with apo-horseradish peroxidase.
**Figure 4** shows the time dependent changes in absorption of assay solutions composed of 0.43 mM TMB and 0.17 mM hydrogen peroxide in 0.1 M Tris buffer (pH 8.8) upon injection of samples containing following components in 0.1 M Tris buffer: a) 1 mM apo-horseradish peroxidase, 32 mM hemin di(N,N'-acetyl-hydrazide) pre-incubated for 10 min; b) 1 mM apo peroxidase, 32 mM hemin di(N,N'-acetylhydrazide), 0.17 mM H₂O₂ pre-incubated for 10 min; c) 1 mM apo peroxidase, 32 mM hemin di(N,N'-acetyl-hydrazide), 0.17 mM H₂O₂ pre-incubated for 5 s; d) 32 mM hemin di(N,N'-acetyl-hydrazide), 0.17 mM H₂O₂ pre-incubated for 10 min; e) 1 mM apo peroxidase, 0.17 mM H₂O₂ pre-incubated for 10 min; f) 32µM hemin di(N,N'-acetyl-hydrazide, 0.17 mM H₂O₂ preincubated for 10 min, and 1 mM apo-horseradish peroxidase added just before the activity assay.

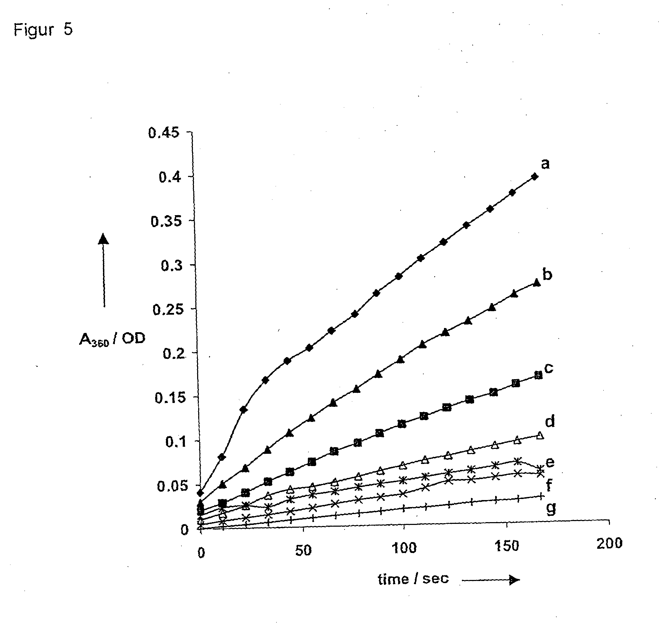

**Figure 5** shows the time dependent changes in absorption of assay solutions composed of 1 mM TMB and 2.3 mM hydrogen peroxide in 0.2 M sodium citrate buffer (pH 4.0) upon injection of samples containing 110 µM EDTA, 0.26 M NaCl, 0.86 µM apo-horseradish peroxidase, 2.33 µM hemin di(N,N'-acetyl-hydrazide) in 0.1 M Tris buffer (pH 7.5) and varying concentrations of H₂O₂: a) 1600 nM; b) 800 nM; c) 400 nM; d) 200 nM; e) 100 nM; f) 50 nM; g) 0 nM.

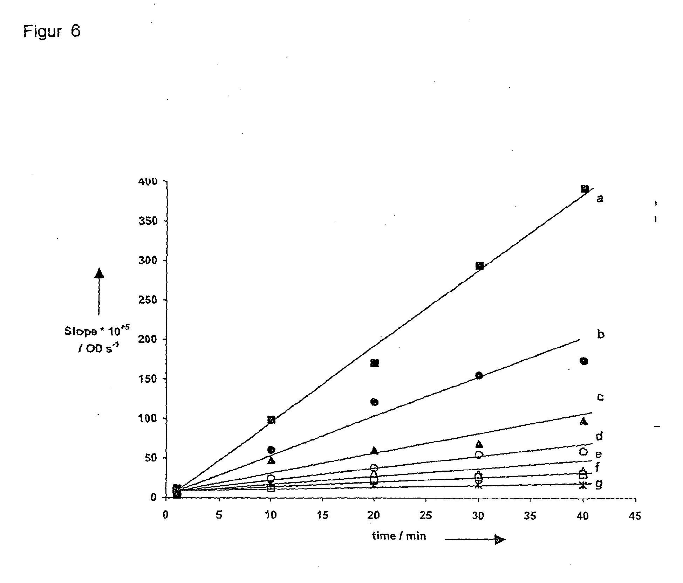

**Figure 6** shows the development of generated peroxidase activity with time for the following concentrations of apo-horseradish peroxidase: a) 1600 nM; b) 800 nM; c) 400 nM; d) 200 nM; e) 100 nM; f) 50 nM; g) 0 nM. Experimental conditions are the same as in Figure 5.

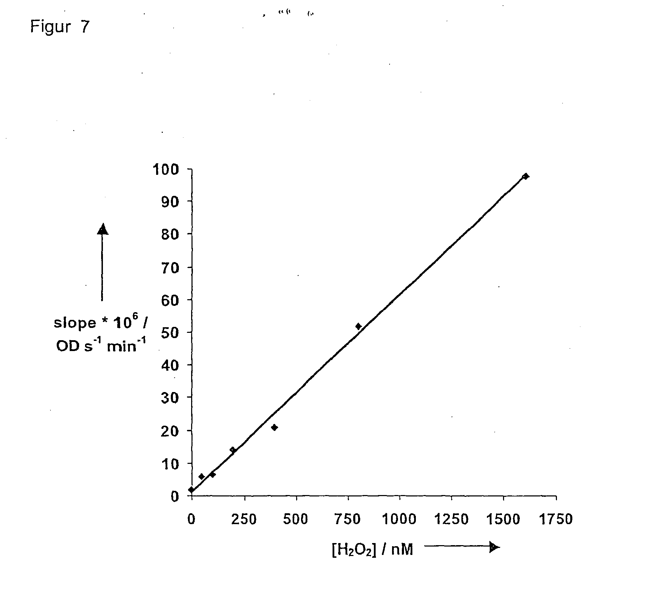

**Figure 7** shows the effect of H₂O₂ concentration during pre-incubation with hemin di(N,N'-acetyl-hydrazide) and apo-horseradish peroxidase on the rate of peroxidase generation. Experimental conditions are the same as in Figure 5.
**Figure 8** shows the time dependent changes in absorption of assay solutions composed of 1 mM TMB and 2.3 mM hydrogen peroxide in 0.2 M sodium citrate buffer (pH 4.0) upon injection of samples containing 110 µM EDTA, 0.26 M NaCl, 0.86 µM apo-horseradish peroxidase, 2.33 pM hemin di(N,N'-acetyl-hydrazide), 72 mM D-glucose in 0.1 M Tris buffer (pH 7.5) and varying concentrations of glucose oxidase: a) 3700 µU/mL; b) 377 µU/mL; c) 37 µU/mL; d) 0 µU/mL.

## Claims

1. A hemin derivative which has the following structure: 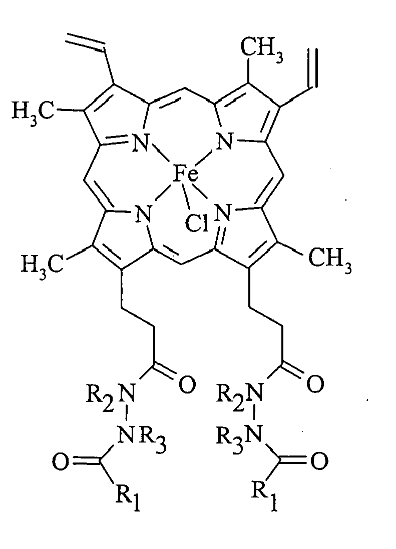 wherein
R₁ represents hydrogen, -ORₐ, -SRₐ, -NRₐR_{b}, a C₁₋₈ straight chain or branched chain alkyl residue, an unsubstituted or C₁₋₈ alkyl- or C₁₋₈ alkoxy-substituted C₆₋₁₀ aryl residue, particularly phenyl or tolyl, or a C₆₋₁₀ aralkyl residue, particularly benzyl, with Rₐ and R_{b} being independently from each other selected from hydrogen, a C₁₋₈ straight chain or branched chain alkyl residue, a C₆₋₁₀ aryl residue, particularly phenyl, or a C₆₋₁₀ aralkyl residue, preferably benzyl, and
R₂ and R₃, independently from each other, represent hydrogen, a C₁₋₈ straight chain or branched chain alkyl residue, a C₆₋₁₀ aryl residue, particularly phenyl, or a C₆₋₁₀ aralkyl residue, preferably benzyl.

2. The hemin derivative according to claim 1, wherein R₁ represents a C₁₋₈ straight chain alkyl residue, preferably methyl.

3. A kit comprising
(i) a hemin derivative according to claim 1 or 2, and
(ii) apo-horseradish peroxidase (apo-HRP).

4. The kit according to claim 3, further comprising:
(iii) a horseradish peroxidase substrate selected from the group consisting of chromogenic, fluorogenic and luminescent substrates.

5. The kit according to claim 4, wherein the horseradish peroxidase substrate is a chromogenic substrate, particularly 3,3',5,5'-tetramethylbenzidine dihydrochloride (TMB).

6. Use of the kit according to claim 4 or 5 for detecting and/or quantifying hydrogen peroxide in a biological sample.

7. An assay method for detecting and/or quantifying an analyte in a biological sample which comprises detecting hydrogen peroxide generated in the presence as opposed to the absence of said analyte by bringing said sample into contact with the kit according to claim 4 or 5.

8. The method according to claim 7, wherein the hydrogen peroxide to be detected is produced *in situ* in the sample by the action of an oxidase on an oxidase substrate contained in said sample.

9. The method according to claim 8 wherein said oxidase substrate which is subjected to the action of the oxidase is selected from glucose and cholesterol.
